# EUROPEAN PATENT SPECIFICATION

(11) **EP 1 372 677 B1**
(45) Date of publication and mention of the grant of the patent: **30.04.2008**
(21) Application number: 02708107.4
(22) Date of filing: 28.03.2002
(51) Int. Cl.: A61K 31/015, A61P 35/00, A61K 36/00

(54) **USE OF COMPOSITIONS COMPRISING SESQUITERPENE DERIVATIVES FOR THE TREATMENT OF CANCER**
VERWENDUNG VON SESQUITERPENE ENTHALTENDEN ZUSAMMENSTELLUNGEN ZUR BEHANDLUNG VON KREBS
UTILISATION DES COMPOSITIONS COMPORTANT DES DERIVES SESQUITERPENIQUES POUR LE TRAITEMENT DE CANCER

(30) Priority: 28.03.2001 CA 2342403
(43) Date of publication of application: 02.01.2004
(73) Proprietor: F.P.L. Pharma Inc., Montréal Québec H4C 2C7 (CA)
(72) Inventor: PICHETTE, André, Chicoutimi, Québec G7H 4L9 (CA); LEGAULT, Jean, Laterriere, Québec G7N 1B6 (CA); MADELMONT, Jean-Claude, F-63540 France (FR)
(74) Representative: Peaucelle, Chantal
(86) International application number: PCT/CA2002/000440
(87) International publication number: WO 2002/078719

(56) References cited:
- FR-A- 2 808 196
- FR-A- 2 811 899
- GB-A- 395 007
- DATABASE EPODOC [Online] EUROPEAN PATENT OFFICE, THE HAGUE, NL; XP002203258 & CN 1 130 062 A (YANG DEPO) 4 September 1996 (1996-09-04)
- MURAKAMI A ET AL: "Identification of zerumbone in Zingiber zerumbet Smith as a potent inhibitor of 12-O-tetradecanoylphorbol-13-acetate-induc ed Epstein-Barr virus activation." BIOSCIENCE, BIOTECHNOLOGY, AND BIOCHEMISTRY. JAPAN OCT 1999, vol. 63, no. 10, October 1999 (1999-10), pages 1811-1812, XP001073951 ISSN: 0916-8451
- KUBO I ET AL: "Cytotoxic and antioxidative sesquiterpenoids from Heterotheca inuloides." PLANTA MEDICA. GERMANY OCT 1996, vol. 62, no. 5, October 1996 (1996-10), pages 427-430, XP008004655 ISSN: 0032-0943
- KANEDA N ET AL: "Plant anticancer agents, L. cytotoxic triterpenes from Sandoricum koetjape stems." JOURNAL OF NATURAL PRODUCTS. UNITED STATES MAY 1992, vol. 55, no. 5, May 1992 (1992-05), pages 654-659, XP008004656 ISSN: 0163-3864
- ZHENG G.-Q.; KENNEY P.M.; LAM L.K.T.: 'Sesquiterpenes from clove (Eugenia Caryophyllata) as potential anticarcinogenic agents' JOURNAL OF NATURAL PRODUCTS vol. 55, no. 7, July 1992, pages 999 - 1003

## Description

### BACKGROUND OF THE INVENTION

### (a) Field of the Invention

The present invention relates to novel antitumor compositions, and the use of sesquiterpene derivatives as antitumor agents.

### (b) Description of Prior Art

To date, antitumor activity of α-humulene and isocaryophyllene (y-caryophyllene) has never been reported in the literature including scientific journals and patents. However, recently Rauter et al. shows a cytotoxic activity of some new humulene derivatives isolated from *Asteriscus vogelii* (Rauter, A.P. et al.; Phytochemistry, 56, 167-171 (2001). These derivatives include the 8-oxo-α-humula-6Z,9Z-dien-12-oic acid, the 8-oxo-α-humula-6E,9Z-dien-12-oic acid and the 8-oxo-α-humula-6E,9E-dien-12-oic acid. Moreover, an anticarcinogenic activity for β-caryophyllene, β-caryophyllene oxide and α-humulene has been reported by Zheng et al. in 1992 (Zheng, G-Q. et al.; Journal of Natural Products, 55, 999-1003 (1992)). It is important to clarify that anticarcinogenic indicates an inhibition of the carcinogenesis process induced by various chemical agents. The research of Zheng et al. is limited to use these compounds in the prevention of cancers. Indeed, Zheng et al. shows *in vivo,* an increasing of gluthatione-S-transferase activity induced by β-caryophyllene, β-caryophyllene oxide and α-humulene. Glutathione-S-transferase is an enzyme responsible of the detoxification of various toxic xenobiotics including the anticarcinogenic agents. Although Zheng et al. suggest that the increasing of glutathione-S-transferase activity could prevent the formation of cancers, they do not demonstrate this hypothesis and do not investigate the antitumoral activity of these compounds.

Antitumoral activity of β-caryophyllene and β-caryophyllene oxide against some solid tumors has been reported by Kubo et al. in Planta Medica (Kubo, I. et al.; planta Medica, 62, 427-430, (1996)). The antitumoral activity of these two compounds was first evaluated *in vitro* by the Applicants, but no significant bioactivity for β-caryophyllene and β-caryophyllene oxide was found. Moreover, an other group shows that β-caryophyllene oxide is inactive *in vitro* against about ten tumor cell lines (Kaneda, N. et al.; Journal of Natural Products, 55, 654-659, (1992)). Finally, Tambe et al., in 1996, demonstrated an gastric cytoprotection effect for β-caryophyllene by inhibition of gastric mucosal injuries induced by necrotizing agents such as absolute ethanol and HCl (Tambe, Y. et al.; Planta Medica. 62, 469-470).

It would be highly desirable to be provided with novel antitumor compositions, the use of sesquiterpene derivatives as antitumor agents and to methods of treatment thereof.

### SUMMARY OF THE INVENTION

One aim of the present invention is to provide novel antitumor compositions, and the use of sesquiterpene derivatives as antitumor agents.

In accordance with the present invention, there is provided an antitumor composition comprising a therapeutically effective quantity of at least one sesquiterpene derivative in association with a pharmaceutically acceptable carrier.

The sesquiterpene derivative may be extracted from a vegetal oil, including, *Pinus banksiana* oil, *Pinus divaricata* oil, *Pinus strobus* oil, *Pinus resinosa* oil, *Pinus sylvestris* oil, *Picea glauca* oil, *Picea marina* oil, *Thuya occidentalis* oil, *Abies balsamea* oil and *Myrica gale* oil.

The sesquiterpene derivative of the present invention includes, isocaryophyllene and α-humulene.

A preferred antitumor composition of the present invention comprises a therapeutically effective quantity of at least a first sesquiterpene derivative and of a second compound selected from the group consisting of a second sesquiterpene derivative being different than a first derivative, an anti-viral agent, an anti-cancer agent, an immunosuppressive agent, and an anti-inflammatory agent.

Preferably, the antitumor composition of the present invention comprises isocaryophyllene or α-humulene as a first sesquiterpene derivative and a second sesquiterpene derivative selected from the group consisting of isocaryophyllene and α-humulene, said second compound being different than the first derivative.

In accordance with the present invention, there is provided the use of the antitumor composition of the present invention for the preparation of a medicament for reducing tumor growth.

In accordance with the present invention, there is provided the use of the antitumor composition of the present invention for the preparation of a medicament for treatment of a tumor.

In accordance with the present invention, there is provided a method for reducing tumor growth comprising administering a therapeutically effective amount of the antitumor composition of the present invention to a patient.

In accordance with the present invention, there is provided a method for treating a tumor in a patient, said method comprising administering a therapeutically effective amount of the antitumor composition of the present invention to a patient.

For the purpose of the present invention the following terms are defined below.

The term "sesquiterpene derivative" is intended to mean one of isocaryophyllene (γ-Caryophyllene), and α-humulene.

The term "tumor" is intended to mean any type of tumor of a cell selected from the group of breast, prostatic, lung, bronchi, gastrointestinal tract, stomach, colon, skin, kidney, brain, leukemic, liver, pancreatic, bone and joint, central nervous system, peripheral nervous system, heart, gonad (ovary), genitourinary, esophageal, pharynx, neuroendocrine, nose and paranasale sinus, and inner ear. The tumor may be benign or malignant. The tumor is intended to include, carcinoma, neoplasm, neuroma, carcinoid, blastoma, adenocarcinoma, and melanoma.

The term "pharmaceutically acceptable carrier" is intended to mean any suitable carrier or adjuvant for any mode of administration for providing a mammal, especially a human, with an effective dosage of the antitumor composition of the present invention. For example, mode of administration, includes without limitation, oral, intravenous, intramuscular, subcutaneous, transdermal, parenteral and topical. Dosage forms include, without limitation, tablets, capsules, powders, solutions, dispersions, suspensions, creams, ointments and aerosols.

The term "patient" is intended to mean any mammal patient, which includes, human, canine, bovine, porcine, murine, caprine, ovine, feline, and equine.

### BRIEF DESCRIPTION OF THE DRAWINGS

Fig. 1A illustrates the molecular structure of α-humulene;
Fig. 1B illustrates the molecular structure of β-Caryophyllene;
Fig. 1C illustrates the molecular structure of γ-Caryophyllene;
Fig. 1D illustrates the molecular structure of Caryophyllene oxide;
Fig. 2 illustrates the evaluation of antitumor activity of essential oil of pine on murine L1210 tumor cells implanted in B6D2F1 mice; and
Fig. 3 illustrates the evaluation of antitumor activity of β-caryophyllene + humulene compared to β-caryophyllene + isocaryophyllene on murine L1210 tumor cells implanted in B6D2F1 mice.

### DETAILED DESCRIPTION OF THE INVENTION

Surprisingly and in accordance with the present invention, there is provided *in vivo* evidence supporting the usefulness of the present antitumor composition.

### Cell culture

Normal human fibroblasts were purchased from Biopredic International (Rennes, France). This frozen culture was obtained from a 36-years old female abdominal surgical waste and the cells used in this work was from the 7^{th} to the 12^{th} passage of the culture. M4Beu and M3DAU, human melanoma cell lines, was established in the laboratory of Dr. J. F. Doré (Institut National de la Santé et de la Recherche Médicale-INSERM, Unit 218, Lyon, France) from metastatic biopsy specimens (Thomasset, N. et al.; British Journal of Cancer, 46, 58-66 (1982)). Murine colon carcinoma cell line CT-26 was obtained from Dr I.J. Fidler (University of Texas M.D. Anderson Cancer Center, Houston). Breast cancer adenocarcinoma MCF-7 and MDA-MB-231, prostatic adenocarcinoma PC-3, ovary adenocarcinoma SK-OV-3, lung carcinoma A-549, colon adenocarcinoma DLD-1, human cell lines and melanoma B16 and fibroblast L-929 murine cell lines were purchased from American Type of Culture Collection (ATCC) or the European Collection of Cell Cultures (ECACC; Salisbury, United-Kingdom). Stock cell cultures were maintained as monolayers in 75-cm² culture flasks in Glutamax^{™} Eagle's minimum essential medium with Earle's salts (Gibco-BRL, Paisley, Scotland) supplemented with 10% foetal calf serum (Sigma), and 5 ml of a 100X solution of vitamins (Gibco), 5 ml of 100 mM sodium pyruvate (Gibco), 5 ml of 100X non-essential amino acids (Gibco) and 2mg of gentamicin base (Gibco). Cells were grown in a humidified 37°C incubator containing 5% CO₂.

### In vitro survival assays

Cells were plated at a density of 5 x 10³ cells per well in 96-well microplates (Nunclon^{™}, Nunc) in 100 µl of culture medium and were allowed to adhere for 16 h before treatment. Then, 100 µl of culture medium containing essential oil of *Pinus divaricata,* essential oil of *Thuya occidentalis,* essential oil of *Abies balsamea,* α-humulene (Sigma-Aldrich); isocaryophyllene (Sigma-Aldrich) were added and incubated at 37°C for 48 h. All compounds were dissolved in ethanol and the final concentration of ethanol in the culture medium was maintained at 0.5 % (v/v). The effect of essential oils and compounds of oil on the proliferation of tumour cells was assessed using resazurin reduction test.

### Resazurin reduction test

Resazurin was recently identified as the alamar Blue dye (O'Brien, J. et al.; Europeen Journal of Biochemistry, 267, 5421-6, (2000)). The resazurin reduction test (RRT) was carried out according to the protocol described here. Briefly, plates were rinsed by 200 µl PBS (37°C, Gibco) at 37°C using an automatic microplate washer (Cell Wash^{™}, Labsystems, Helsinki, Finland) and emptied by overturning on absorbent toweling. Then, 150 µl of a 25 µg/ml solution of resazurin in RPMI 1640 without Phenol red was added in each well using an automatic microvolume dispenser (Multidrop 384^{™} Labsystems). The plates were incubated 1 h at 37°C in an humidified atmosphere with 5% of CO₂ for fluorescence development by living cells. Fluorescence was then measured on the automated 96-well plate reader Fluoroskan Ascent FL^{™} (Labsystems) using an excitation wavelength of 530 nm and an emission one of 590 nm. The fluorescence is proportional to the number of living cells in the well and IC₅₀ (drug concentration required to inhibit cell proliferation by 50%) was calculated from the curve of concentration-dependent survival percentage, defined as the fluorescence in experimental wells as a percentage of that in control wells, with blank values subtracted.

### Evaluation of antitumor activity in mice

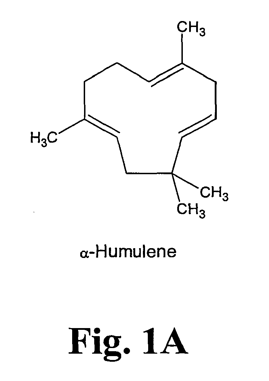

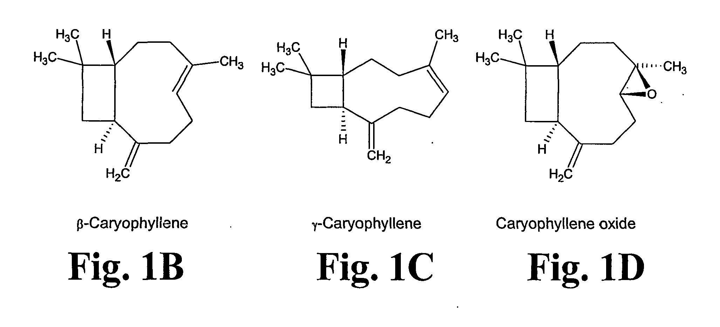

B6D2F1 male mice, 18-20 g, were obtained from Iffa Credo (Lyon, France). Murine leukemia cell line, L1210 (1×10⁵), were implanted intraperitoneally on day 0. The treatment were administered intraperitoneally on a day -1, -5 and -9 schedule and was delivered as one injection of 0.2 ml/25 g mouse body weight. Each experimental group contained six animals. Each agent including essential pine oil, β-caryophyllene (Fig. 1B), α-humulene (Fig. 1A) and isocaryophyllene (γ-caryophyllene, Fig. 1C)were administered at 1000 mg/kg.

### Identification of bioactive natural products and results of biological activity studies

The essentials oil of jack pine (*Pinus banksiana* or *Pinus divaricata*), eastern white cedar (*Thuya occidentalis*), balsam fir (*Abies balsamea*), were evaluated for their cytotoxic activity against various tumor cell lines (Table 1). The cell growth inhibition were measured by the fluorescence induced by the metabolic transformation of resazurin in resorufin. The fluorescence is proportional to living cells and allow to evaluate the concentration inhibiting 50 % of cell growth (IC₅₀). Several essential oils have demonstrated a cytotoxic activity against all tumor cell lines tested including : breast adenocarcinoma MCF-7 and MDA-MB-231, prostatic adenocarcinoma PC-3, ovary adenocarcinoma SK-OV-3, lung carcinoma A-549, colon adenocarcinoma DLD-1 and melanoma M4BEU and M3DAU.

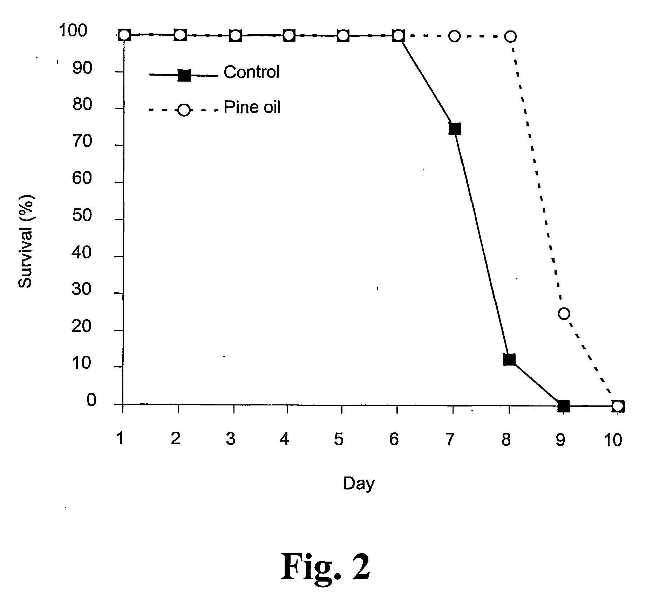

Cytotoxic activity of sesquiterpene oil constituent was evaluated against various tumor cell lines in order to identify bioactive compounds. A sesquiterpene of the humulane family, α-humulene (also known as α-caryophyllene, 2,6,6,9-Tetramethyl-1,4,8-cycloundecatriene) (Fig.2), is a compound of essential oil demonstrating a significant activity against tumor cells (Table 2). Indeed, α-humulene is active (< 100 µmol) against breast adenocarcinoma MCF-7, prostatic adenocarcinoma PC-3, lung carcinoma A-549, colon adenocarcinoma DLD-1 and melanoma M4BEU (Table 2).

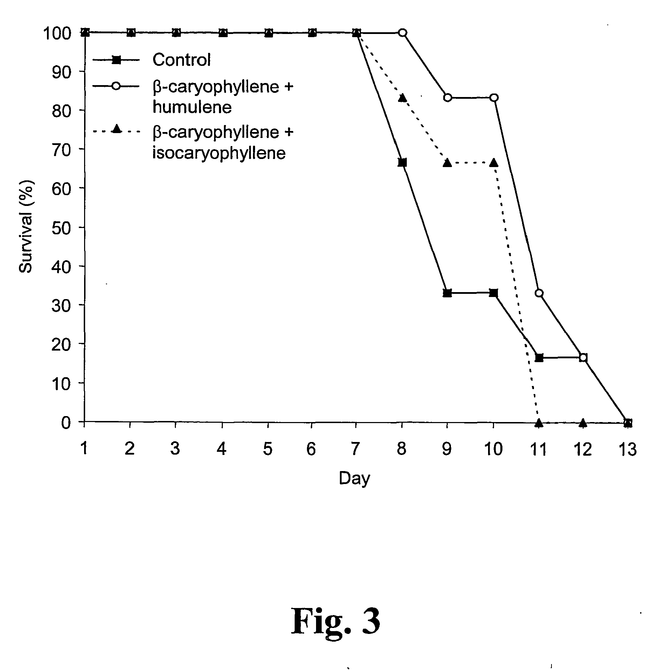

Cytotoxic activity of a sesquiterpene related to α-humulene (Fig. 3), isocaryophyllene, have been evaluated. Isomer *cis* of β-caryophyllene or isocaryophyllene (also known as γ-caryophyllene, Z-caryophyllene, cis-4,11,11-Trimethyl-8-methylenebicyclo[7.2.0] undec-4-ene) demonstrated as α-humulene, a cytotoxic activity (< 100 µmol, Table 2) against all tumor cells tested. Moreover, isocaryophyllene shown to be slightly less active against fibroblastic normal cell in comparison to tumor cells (> 100 µmol, Table 2).

Antitumor activity of essential oil of pine on murine L1210 tumor cells implanted in B6D2F1 mice was evaluated. In Fig. 2, the results show that the essential oil of pine increase the survival of mice. Moreover, the antitumor activity of sesquiterpene including β-caryophyllene in combination with humulene and β-caryophyllene in combination with isocaryophyllene was evaluated *in vivo.* The results appearing in Fig. 3 shows that β-caryophyllene with humulene as well as β-caryophyllene with isocaryophyllene resulted in an increased life span.

**Table 1**

| **Cytotoxic activity of essential oil *of Pinus divaricata, Thuya occidentalis* and *Abies balsamea* against various tumor cell lines** | | | | |
|---|---|---|---|---|
| | | IC₅₀ (% v,v)^{a} | | |
| Tissue | Cell lines | *Pinus divaricata* | *Thuya occidentalis* | *Abides balsamea* |
| human breast adenocarcinoma | MCF-7^{c,d} | 0.11 ± 0.02^{b} | 0.12 ± 0.03^{b} | 0.15 ± 0.07^{b} |
| human breast adenocarcinoma | MDA-MB-231^{c,d} | 0.067 ± 0.003^{b} | 0.070 ± 0.004^{b} | 0.20 ± 0.01^{b} |
| human prostatic adenocarcinoma | PC-3^{d,e} | ND^{c} | ND^{c} | 0.19 ± 0.04^{b} |
| human ovary adenocarcinoma | SK-OV-3^{e} | 0.038 ± 0.002 | 0.043 ± 0.002 | 0.10 ± 0.01^{b} |
| human lung carcinoma | A-549^{d,e} | 0.028 ± 0.001 | 0.086 ± 0.003 | 0.13 ± 0.03^{b} |
| human colon adenocarcinoma | DLD-1^{d,e} | ND^{c} | ND^{c} | 0.16 ± 0.03^{b} |
| human melanoma | M4BEU^{f} | 0.047 ± 0.004 | 0.11 ± 0.01 | 0.14 ± 0.03^{b} |
| human melanoma | M3DAU^{f} | 0.064 ± 0.04 | 0.20 ± 0.02 | 0.22 ± 0.07^{b} |
| human fibroblast | Fibroblast^{g} | 0.07 ± 0.02 | 0.16 ± 0.03 | 0.24 ± 0.06^{b} |
| mouse melanoma | B16-F0^{d,e} | ND^{c} | ND^{c} | 0.09 ± 0.02^{b} |
| mouse subcutaneous connective tissue | L-929^{d,e} | 0.035 ± 0.08 | 0.107 ± 0.008 | 0.18 ± 0.01^{b} |
| mouse colon carcinoma | CT-26^{h} | 0.054 ± 0.002 | 0.061 ± 0.004 | 0.087±0.004^{b} |

| | | | | |
|---|---|---|---|---|
| *^{a}* Concentration inhibiting cell growth by 50%. *^{b}* Values are mean ± SD of three determinations. *^{c}* ND : not determined *^{d}* ATCC : American Type Culture Collection. *^{e}* ECACC (Salisbury, United-Kingdom) : European Collection of Cell Culture. *^{f}* Thomasset N, Quash G, Dore JF. (1982). Diamine oxidase activity in human melanoma cell lines with different tumorigenicity in nude mice. Br. J. Cancer. Jul;46(1): 58-66. *^{g}* Biopredic International (Rennes, France). *^{h}* Brattain MG, Strobel-Stevens J, Fine D, Webb M, Sarrif AM. (1980). Establishment of mouse colonic carcinoma cell lines with different metastatic properties. Cancer Res. Jul;40(7): 2142-6. | | | | |

**Table 2**

| **Cytotoxic activity of sesquiterpenes on various tumor cell lines** | | |
|---|---|---|
| | **IC₅₀ (µM)** | |
| **Cells lines** | **Isocaryophyllene** | **α-humulene** |
| MCF-7 | 84 ± 6^{b} | 73 ± 2^{b} |
| PC-3 | 87 ± 8^{b} | 73 ± 2^{b} |
| A-549 | 59 ± 4^{b} | 68 ± 2^{b} |
| DLD-1 | 124 ± 17^{b} | 71 ± 2^{b} |
| M4BEU | 43 ± 3^{b} | 55 ± 2^{b} |
| Fibroblast | 124 ± 15^{b} | 85 ± 5^{b} |
| L-929 | 34.0 ± 0.8^{b} | 50.2 ± 0.3^{b} |
| CT-26 | 46 ± 1^{b} | 53 ± 1^{b} |

| | | |
|---|---|---|
| *^{a}* Concentration inhibiting cell growth by 50%. *^{b}* Values are mean ± SD of three determinations. | | |

## Claims

1. Use of an antitumor composition against solid tumors comprising a therapeutically effective quantity of at least one sesquiterpene derivative selected from the group consisting of isocaryophyllene and α-humulene in association with a pharmaceutically acceptable Carrier, for the preparation of a medicament for reducing tumor growth.

2. The use of claim 1, wherein said derivative is extracted from a vegetal oil.

3. The use of claim 2, wherein said vegetal oil is selected from the group consisting of *Pinus banksiana* oil, *Pinus divaricata* oil, *Pinus strobus* oil, *Pinus resinosa* oil, *Pinus sylvestris* oil, *Picea glauca* oil, *Picea mariana* oil, *Thuya occidentalis* oil, *Abies balsamea* oil and *Myrica gale* oil.

4. The use of claim 1, wherein said composition further comprises a therapeutically effective quantity of at least a second compound selected from the group consisting of isocaryophyllene and α-humulene said second compound being different than a first derivative; an anti-cancer agent, an immunosuppressive agent, and an anti-inflammatory agent.

5. The use of claim 1, wherein said tumor is of a cell selected from the group of breast, prostatic, lung, bronchi, gastrointestinal tract, stomach, colon, skin, kidney, brain, leukemic, liver, pancreatic, bone and joint, central nervous system, peripheral nervous system, heart, gonad, genitourinary, esophageal, pharynx, neuroendocrine, nose and paranasale sinus, and inner ear.

6. The use of claim 5, wherein said tumor is selected from the group of carcinoma, neoplasm, neuroma, carcinoid, blastoma, adenocarcinoma, and melanoma.

7. Us of an antitumor composition against solid tumors comprising a therapeutically effective quantity of at least one sesquiterpene derivative selected from the group consisting of isocaryophyllene and α-humulene in association with a pharmaceutically acceptable carrier, for the preparation of a medicament for treating a tumor in a patient.

8. The use of claim 7, wherein said tumor is of a cell selected from the group of breast, prostatic, lung, bronchi, gastrointestinal tract, stomach, colon, skin, kidney, brain, leukemic, liver, pancreatic, bone and joint, central nervous system, peripheral nervous system, heart, gonad, genitourinary, esophageal, pharynx, neuroendocrine, nose and paranasale sinus, and inner ear.

9. The use of claim 7, wherein said tumor is selected from the group of carcinoma, neoplasm, neuroma, carcinoid, blastoma, adenocarcinoma, and melanoma.

10. The use of claim 7, wherein said derivative is extracted from a vegetal oil.

11. The use of claim 7, wherein said vegetal oil is selected from the group consisting of *Pinus banksiana* oil, *Pinus divaricate* oil, *Pinus strobus* oil, *Pinus resinosa* oil, *Pinus sylvestris* oil, *Picea glauca* oil, *Picea mariana* oil, *Thuya occidentalis* oil, *Abies balsamea* oil and *Myrica gale* oil.

12. The use of claim 7, wherein said composition further comprises a therapeutically effective quantity of at least a second compound selected from the group consisting of isocaryophyllene and α-humulene said second compound being different than a first derivative; an anti-cancer agent, an immunosuppressive agent, and an anti-inflammatory agent.

## Patentansprüche

1. Verwendung einer Antitumorzusammensetzung gegen feste Tumoren, die eine therapeutisch wirksame Menge wenigstens eines Sesquiterpen-Derivats, das aus der Gruppe ausgewählt ist, die aus Isocaryophyllen und α-Humulen besteht, in Verbindung mit einem pharmazeutisch annehmbaren Träger umfasst, zur Herstellung eines Medikaments zum Reduzieren von Tumorwachstum.

2. Verwendung gemäß Anspruch 1, wobei das Derivat aus einem Pflanzenöl extrahiert ist.

3. Verwendung gemäß Anspruch 2, wobei das Pflanzenöl aus der Gruppe ausgewählt ist, die aus *Pinus-banksiana*-Öl, *Pinus-divaricata*-Öl*, Pinusstrobus*-Öl*, Pinus-resinosa-*Öl*, Pinus-sylvestris*-Öl*, Picea-glauca*-Öl*, Piceamariana*-Öl*, Thuya-occidentalis*-Öl*, Abies-balsamea*-Öl und *Myrica-gale*-Öl besteht.

4. Verwendung gemäß Anspruch 1, wobei die Zusammensetzung weiterhin Folgendes umfasst: eine therapeutisch wirksame Menge wenigstens einer zweiten Verbindung, die aus der Gruppe ausgewählt ist, die aus Isocaryophyllen und α-Humulen besteht, wobei die zweite Verbindung von dem ersten Derivat verschieden ist; ein Krebsmedikament, ein immunsuppressives Mittel und ein entzündungshemmendes Mittel.

5. Verwendung gemäß Anspruch 1, wobei der Tumor auf einer Zelle eines Gewebes beruht, das aus der Gruppe ausgewählt ist, die aus Brust, Prostata, Lunge, Bronchien, Magen-Darm-Trakt, Magen, Dickdarm, Haut, Niere, Gehirn, leukämischen Zellen, Leber, Pankreas, Knochen und Gelenk, Zentralnervensystem, peripherem Nervensystem, Herz, Keimdrüsen, Urogenitalgewebe, Speiseröhre, Pharynx, neuroendokrinem System, Nase und Nasennebenhöhlen sowie Innenohr besteht.

6. Verwendung gemäß Anspruch 5, wobei der Tumor aus der Gruppe ausgewählt ist, die aus Karzinom, Neoplasma, Neurom, Karzinoid, Blastom, Adenokarzinom und Melanom besteht.

7. Verwendung einer Antitumorzusammensetzung gegen feste Tumoren, die eine therapeutisch wirksame Menge wenigstens eines Sesquiterpen-Derivats, das aus der Gruppe ausgewählt ist, die aus Isocaryophyllen und α-Humulen besteht, in Verbindung mit einem pharmazeutisch annehmbaren Träger umfasst, zur Herstellung eines Medikaments zur Behandlung eines Tumors bei einem Patienten.

8. Verwendung gemäß Anspruch 7, wobei der Tumor auf einer Zelle eines Gewebes beruht, das aus der Gruppe ausgewählt ist, die aus Brust, Prostata, Lunge, Bronchien, Magen-Darm-Trakt, Magen, Dickdarm, Haut, Niere, Gehirn, leukämischen Zellen, Leber, Pankreas, Knochen und Gelenk, Zentralnervensystem, peripherem Nervensystem, Herz, Keimdrüsen, Urogenitalgewebe, Speiseröhre, Pharynx, neuroendokrinem System, Nase und Nasennebenhöhlen sowie Innenohr besteht.

9. Verwendung gemäß Anspruch 7, wobei der Tumor aus der Gruppe ausgewählt ist, die aus Karzinom, Neoplasma, Neurom, Karzinoid, Blastom, Adenokarzinom und Melanom besteht.

10. Verwendung gemäß Anspruch 7, wobei das Derivat aus einem Pflanzenöl extrahiert ist.

11. Verwendung gemäß Anspruch 7, wobei das Pflanzenöl aus der Gruppe ausgewählt ist, die aus *Pinus-banksiana*-Öl*, Pinus-divaricata*-Öl*, Pinusstrobus*-Öl*, Pinus-resinosa*-Öl*, Pinus-sylvestris*-Öl*, Picea-glauca*-Öl*, Piceamariana*-Öl*, Thuya-occidentalis*-Öl*, Abies-balsamea*-Öl und *Myrica-gale*-Öl besteht.

12. Verwendung gemäß Anspruch 7, wobei die Zusammensetzung weiterhin Folgendes umfasst: eine therapeutisch wirksame Menge wenigstens einer zweiten Verbindung, die aus der Gruppe ausgewählt ist, die aus Isocaryophyllen und α-Humulen besteht, wobei die zweite Verbindung von dem ersten Derivat verschieden ist; ein Krebsmedikament, ein immunsuppressives Mittel und ein entzündungshemmendes Mittel.

## Revendications

1. Utilisation d'une composition antitumorale contre des tumeurs solides comprenant une quantité efficace sur le plan thérapeutique d'au moins un dérivé sesquiterpénique choisi dans le groupe constitué par l'isocaryophyllène et l'α-humulène en association avec un véhicule acceptable sur le plan pharmaceutique, pour la préparation d'un médicament destiné à réduire le développement de tumeurs.

2. Utilisation selon la revendication 1, dans laquelle ledit dérivé est extrait à partir d'une huile végétale.

3. Utilisation selon la revendication 2, dans laquelle ladite huile végétale est choisie dans le groupe constitué par l'huile de *Pinus banksiana,* l'huile de *Pinus divaricata,* l'huile de *Pinus strobus,* l'huile de *Pinus resinosa,* l'huile de *Pinus sylvestris,* l'huile de *Picea glauca*, l'huile de *Picea mariana*, l'huile de *Thuya occidentalis*, l'huile de *Abies balsamea* et l'huile de *Myrica gale.*

4. Utilisation selon la revendication 1, dans laquelle ladite composition comprend en outre une quantité efficace sur le plan thérapeutique d'au moins un deuxième composé choisi dans le groupe constitué par l'isocaryophyllène et l'α-humulène, ledit deuxième composé étant différent d'un premier dérivé ; un agent anticancéreux, un agent immunosuppresseur et un agent anti-inflammatoire.

5. Utilisation selon la revendication 1, dans laquelle ladite tumeur est formée d'une cellule choisie dans le groupe constitué par le sein, la prostate, les poumons, les bronches, le tractus gastrointestinal, l'estomac, le côlon, la peau, le rein, le cerveau, les cellules sanguines, le foie, le pancréas, les os et articulations, le système nerveux central, le système nerveux périphérique, le coeur, la gonade, le système uro-génital, l'oesophage, le pharynx, la neuroendocrine, le nez et sinus paranasal et l'oreille interne.

6. Utilisation selon la revendication 5, dans laquelle ladite tumeur est choisie dans le groupe constitué par le carcinome, le néoplasme, le névrome, le carcinoïde, le blastocytome, l'adénocarcinome et le mélanome.

7. Utilisation d'une composition antitumorale contre des tumeurs solides comprenant une quantité efficace sur le plan thérapeutique d'au moins un dérivé sesquiterpénique choisi dans le groupe constitué par l'isocaryophyllène et l'α-humulène en association avec un véhicule acceptable sur le plan pharmaceutique, pour la préparation d'un médicament destiné à traiter une tumeur chez un patient.

8. Utilisation selon la revendication 7, dans laquelle ladite tumeur est formée d'une cellule choisie dans le groupe constitué par le sein, la prostate, les poumons, les bronches, le tractus gastrointestinal, l'estomac, le côlon, la peau, le rein, le cerveau, les cellules sanguines, le foie, le pancréas, les os et articulations, le système nerveux central, le système nerveux périphérique, le coeur, la gonade, le système uro-génital, l'oesophage, le pharynx, la neuroendocrine, le nez et sinus paranasal et l'oreille interne.

9. Utilisation selon la revendication 7, dans laquelle ladite tumeur est choisie dans le groupe constitué par le carcinome, le néoplasme, le névrome, le carcinoïde, le blastocytome, l'adénocarcinome et le mélanome.

10. Utilisation selon la revendication 7, dans laquelle ledit dérivé est extrait d'une huile végétale.

11. Utilisation selon la revendication 7, dans laquelle ladite huile végétale est choisie dans le groupe constitué par l'huile de *Pinus banksiana,* l'huile de *Pinus divaricata*, l'huile de *Pinus strobus,* l'huile de *Pinus resinosa*, l'huile de *Pinus sylvestris,* l'huile de *Picea alauca*, l'huile de *Picea mariana*, l'huile de *Thuya occidentalis*, l'huile de *Abies balsamea* et l'huile de *Myrica gale.*

12. Utilisation selon la revendication 7, dans laquelle ladite composition comprend en outre une quantité efficace sur le plan thérapeutique d'au moins un deuxième composé choisi dans le groupe constitué par l'isocaryophyllène et l'α-humulène, ledit deuxième composé étant différent d'un premier dérivé ; un agent anticancéreux, un agent immunosuppresseur et un agent anti-inflammatoire.
